(19) **Europäisches Patentamt / European Patent Office / Office européen des brevets**

(11) **EP 3 365 661 B1**

(12) **FASCICULE DE BREVET EUROPEEN**

(45) Date de publication et mention de la délivrance du brevet:
**26.04.2023 Bulletin 2023/17**

(21) Numéro de dépôt: **16819125.2**

(22) Date de dépôt: **15.10.2016**

(51) Classification Internationale des Brevets (IPC):
**G01N 21/35** [(2014.01)]   **A01K 67/02** [(2006.01)]
**C12N 5/076** [(2010.01)]   **G01N 33/487** [(2006.01)]

(52) Classification Coopérative des Brevets (CPC):
**A01K 67/02; G01N 21/35; G01N 33/487**

(86) Numéro de dépôt international:
**PCT/FR2016/052671**

(87) Numéro de publication internationale:
**WO 2017/068266 (27.04.2017 Gazette 2017/17)**

(54) **PROCEDE DE DETERMINATION DE LA QUALITE D'UNE SEMENCE D'UN ANIMAL VERTEBRE**

VERFAHREN ZUR BESTIMMUNG DER SPERMAQUALITÄT VON WIRBELTIEREN

METHOD FOR DETERMINING THE SPERM QUALITY OF VERTEBRATE ANIMALS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.10.2015 FR 1560026**
**21.07.2016 FR 1656931**

(43) Date de publication de la demande:
**29.08.2018 Bulletin 2018/35**

(73) Titulaire: **SYNETICS FRANCE**
**35538 Noyal Sur Vilaine (FR)**

(72) Inventeurs:
• **GUILLAUME, François**
**35000 Rennes (FR)**
• **LE CORVEC, Maëna**
**35380 Maxent (FR)**
• **DE BRETAGNE, Thomas**
**35310 Breal Sous Montfort (FR)**
• **MICHEL, Gildas**
**35160 Montfort Sur Meu (FR)**
• **BASQUIN, Stéphane**
**35140 Saint Aubin Du Cormier (FR)**
• **CHARREAUX, Frédéric**
**44360 Saint Etienne De Montluc (FR)**
• **TARIEL, Hughes**
**35000 Rennes (FR)**
• **GUYONNET, Benoît**
**35000 Rennes (FR)**

(74) Mandataire: **Ermeneux, Bertrand**
**AVOXA**
**5 allée Ermengarde d'Anjou**
**ZAC Atalante Champeaux**
**CS 40824**
**35108 Rennes (FR)**

(56) Documents cités:
• **DANIEL FILIPE CRUZ ET AL: "Oxidative stress markers: Can they be used to evaluate human sperm quality?", TÜRK ÜROLOJI DERGISI/TURKISH JOURNAL OF UROLOGY, vol. 41, no. 4, 14 octobre 2015 (2015-10-14), pages 198-207, XP055299686, ISSN: 2149-3235, DOI: 10.5152/tud.2015.06887**
• **AMIR ABRAMOVICH ET AL: "Diagnostic and Analysis of Human Sperm Characteristics Using Fourier Transform Infrared Spectroscopy", OPEN JOURNAL OF UROLOGY, vol. 05, no. 06, 1 janvier 2015 (2015-01-01), pages 97-101, XP055299569, ISSN: 2160-5440, DOI: 10.4236/oju.2015.56015**
• **PEREZ-MARIN C C ET AL: "Assessment of porcine semen concentration by near InfraRed Spectroscopy (NIRS)", REPRODUCTION IN DOMESTIC ANIMALS, BLACKWELL WISS. VERLAG, BERLIN, DE, vol. 42, no. Supplement 2, 1 septembre 2007 (2007-09-01), page 92, XP008181483, ISSN: 0936-6768, DOI: 10.1111/J.1439-0531.2007.00910.X [extrait le 2007-08-06]**

EP 3 365 661 B1

- P.B. FARRELL ET AL: "Quantification of bull sperm characteristics measured by computer-assisted sperm analysis (CASA) and the relationship to fertility", THERIOGENOLOGY, vol. 49, no. 4, 1 mars 1998 (1998-03-01), pages 871-879, XP055299859, US ISSN: 0093-691X, DOI: 10.1016/S0093-691X(98)00036-3
- Christensen P ET AL: "The Relationship between Semen Quality and the Nonreturn Rate of Bulls", REPRODUCTION IN DOMESTIC ANIMALS, vol. 34, no. 6, 1 December 1999 (1999-12-01), pages 503-507, XP55843021, DE ISSN: 0936-6768, DOI: 10.1111/j.1439-0531.1999.tb01410.x
- Christensen Preben ET AL: "Relationship Between Sperm Viability as Determined by Flow Cytometry and Nonreturn Rate of Dairy Bulls", JOURNAL OF ANDROLOGY., vol. 26, no. 1, 1 January 2005 (2005-01-01), pages 98-106, XP55843214, US ISSN: 0196-3635, DOI: 10.1002/j.1939-4640.2005.tb02878.x

**Description**

## 1. Domaine de l'invention

L'invention se rapporte au domaine de l'élevage et en particulier à la reproduction du bétail, des aviaires et des poissons.

Plus précisément, l'invention concerne un procédé de détermination de la qualité d'une semence d'un animal vertébré.

L'invention trouve notamment une application dans la sélection d'une semence de qualité permettant d'améliorer le taux de réussite d'une insémination d'un animal dans un élevage ou dans le contrôle de la qualité des semences de reproducteurs.

## 2. État de la technique

Dans le domaine de l'élevage du bétail, l'insémination est une démarche importante pour optimiser la gestion des troupeaux. Cette étape est d'autant plus cruciale qu'elle contribue à maximaliser les périodes de production de lait, par exemple dans le cas des vaches laitières. Pour ces raisons, la qualité de l'insémination et donc de la semence employée est essentielle.

En effet, une insémination qui n'aboutit pas à une gestation de l'animal a une incidence négative sur la rentabilité d'un élevage. Par exemple, pour une vache, le succès d'une insémination est estimé par l'absence d'un retour en oestrus enregistré au cours d'une période de 90 jours après l'acte d'insémination. En cas d'échec, la production annuelle de lait de l'animal concerné est directement impactée à la baisse. Cela se traduit par une baisse de revenu d'exploitation pour l'éleveur. En outre, il faut répéter l'opération d'insémination. Il est donc important de réduire les incertitudes liées à cette intervention.

Ainsi, l'évaluation de la qualité d'une semence est un enjeu considérable pour les laboratoires d'analyse et les producteurs de semence.

Cette qualité a tout d'abord été estimée selon des protocoles de sélection des animaux. Cependant, on a constaté qu'un animal possédant un patrimoine génétique comprenant de très bonnes caractéristiques ne s'avère pas nécessairement être un bon reproducteur. De plus, la qualité de la semence d'un animal sélectionné pour son bon taux de reproduction global peut varier en fonction de la santé de l'animal lors du prélèvement.

On a alors tenté d'évaluer la qualité de la semence prélevée. Cette qualité a été évaluée à partir de la semence dans son ensemble et/ou à partir de ses différents composants en fonction de différents critères.

La semence est un fluide biologique complexe, constitué des sécrétions de différents organes du tractus reproducteurs, dans lesquels baignent les gamètes mâles. La semence est constituée entre autres des spermatozoïdes, du plasma séminal et d'exosomes.

La fertilité qui est l'aptitude à procréer est un terme qui fait consensus pour évaluer la qualité d'une semence. Cependant, ce terme présente l'inconvénient d'être général et vague. Il n'est pas satisfaisant pour effectuer une discrimination des individus mâles. Par conséquent et selon les besoins, différents critères ont été définis afin d'évaluer la fertilité d'un individu.

On trouve parmi ces critères :

- le taux de fécondation qui est le taux de fusion entre les gamètes mâle et femelle permettant la formation du zygote ;
- le TNR, acronyme de taux de non-retour, est une estimation du résultat de l'insémination conclue par un succès ou un échec, basée sur l'absence d'un retour en oestrus enregistré au cours d'un intervalle de X jours après l'acte d'insémination ;
- le taux de conception qui est le pourcentage de femelles diagnostiquées en gestation au cours d'un intervalle de X jours après l'acte d'insémination (DeJarnette JM, Amann RP. « Understanding estimates of AI sire fertility: From A to Z. » 23rd Technical Conference on Artificial Insemination & Reproduction, Milwaukee, WI. 2010).

Une difficulté est qu'une mesure de ces différents critères nécessite, pour obtenir un résultat, une attente plus ou moins longue en fonction des espèces ou des races d'animaux.

De nombreux tests in vitro ont donc été développés pour évaluer la semence dont certains sont recommandés notamment en clinique humaine par l'Organisation mondiale de la Santé (World Health Organization. « WHO laboratory manual for the examination and processing of human semen. » Geneva: World Health Organization; 2010). Dans l'espèce humaine comme pour les autres espèces de vertébrés, les tests existants ne sont pas satisfaisants pour prédire la fertilité d'un échantillon de sperme analysé (De longe C. « Semen analysis: looking for an upgrade in class. » Fertil Steril 2012 - 97:260-266 et Kastelic JP, Thundathil JC. « Breeding soundness évaluation and semen analysis for predicting bull fertility. » Reprod Domest Anim Zuchthyg 2008; 43 Suppl 2:368-373). Les tests macroscopiques (volume, couleur, viscosité...) et microscopiques (motilité, concentration, morphologie...) réalisés actuellement en routine permettent d'éliminer les semences avec des qualités extrêmement médiocres, mais ne sont pas probants pour identifier les infertilités idiopathiques ni les animaux hypofertiles. De plus, ils ne permettent pas, par exemple, de prédire la variabilité des fertilités des taureaux observées sur le terrain. Cette difficulté réside entre autres dans le fait que les spermatozoïdes sont des cellules complexes et que leur évaluation comporte quelques difficultés (Mocé E, Graham JK. « In vitro évaluation of sperm quality. » Anim Reprod Sci 2008; 105:104-118).

Un des problèmes inhérent à l'évaluation de la qualité d'une semence provient du fait que l'échantillon contient une population hétérogène de spermatozoïdes. À la suite de leur formation au cours de la spermatogenèse, les spermatozoïdes traversent l'épididyme et s'accumulent dans la queue de cet organe jusqu'au moment de l'éjaculation (Dacheux J-L, Dacheux F. « New insights into epididymal function in relation to sperm maturation. » Reprod Camb Engl 2014; 147:R27-42). Les spermatozoïdes ainsi stockés proviennent de différentes vagues de spermatogenèse, donc, les spermatozoïdes provenant d'un même éjaculat présentent différents degrés de maturation assurant, in vivo, une plus large fenêtre de fécondation. In vivo au cours de leur transit dans le tractus reproducteur femelle, certains spermatozoïdes sont éliminés de la population. Par contre lors de l'analyse in vitro d'une semence, l'échantillon peut comprendre plusieurs spermatozoïdes infertiles, qui peuvent être immobiles, morts, mal formés, ... (Holt WV, Van Look KJW. « Concepts in sperm heterogeneity, sperm sélection and sperm compétition as biological foundations for laboratory tests of semen quality. » Reprod Camb Engl 2004; 127:527-535 et Rodriguez-Martinez H. « Can we increase the estimated value of semen assessment? » Reprod Domest Anim Zuchthyg 2006; 41 Suppl 2:2-10 et Petrunkina AM, Volker G, Brandt H, Töpfer-Petersen E, Waberski D. « Functional significance of responsiveness to capacitating conditions in boar spermatozoa. » Theriogenology 2005; 64:1766-1782). Ainsi, les spermatozoïdes infertiles sont évalués au même titre que ceux qui seront aptes à féconder l'ovule.

Un autre problème inhérent à l'évaluation de la qualité d'une semence est que les spermatozoïdes ne répondent pas uniformément à un même stress (Petrunkina AM, Volker G, Brandt H, Töpfer-Petersen E, Waberski D. « Functional significance of responsiveness to capacitating conditions in boar spermatozoa. » Theriogenology 2005; 64:1766-1782), même si ces spermatozoïdes présentent des caractéristiques similaires à certains moments spécifiques. Finalement, la complexité de travailler avec des spermatozoïdes vient également du fait que cette cellule est multicompartimentée et que chacun de ces sous-compartiments doit être intact et fonctionnel pour permettre la fécondation ( Amann RP, Hammerstedt RH. « In vitro évaluation of sperm quality: an opinion. » J Androl 1993; 14:397-406).

Pour assurer la fécondation, les spermatozoïdes doivent posséder plusieurs caractéristiques telles qu'une mobilité, une production d'ATP, une induction de l'hyperactivation, une faculté à réaliser leur capacitation et leur réaction acrosomique, une membrane plasmique fonctionnelle, une capacité à reconnaître et à se lier à la zone pellucide, ou encore, posséder un ADN intact, etc. Les spermatozoïdes sont donc des cellules complexes, multifonctionnelles nécessitant le bon fonctionnement de plusieurs paramètres pour atteindre leur but ultime : la fécondation et le soutien du développement embryonnaire précoce.

L'infertilité ou l'hypofertilité d'un individu peut être la conséquence d'une multitude de modifications.

Ainsi, un test évaluant un des paramètres des spermatozoïdes parvient difficilement à détecter un spermatozoïde défectueux pour une propriété autre que celle évaluée par le test. Un inconvénient d'un tel test est de surestimer la fertilité de l'échantillon (Graham JK, Mocé E. « Fertility évaluation of frozen/thawed semen. » Theriogenology 2005; 64:492-504). De plus, il est peu probable que ce test en laboratoire évaluant un paramètre des spermatozoïdes soit en mesure de détecter la proportion de spermatozoïdes contenant l'ensemble des paramètres nécessaires pour féconder l'ovocyte et assurer le développement embryonnaire.

C'est pourquoi les tests multiparamétriques s'avèrent intéressants. En effet, l'analyse de plusieurs paramètres d'un échantillon de spermatozoïdes permet d'obtenir une vision globale de l'échantillon analysé et améliore la détection d'un paramètre déficient. De cette façon, l'analyse multiparamétrique permettrait de mieux expliquer les écarts de fertilité entre les différents taureaux analysés (Januskauskas A, Johannisson A, Söderquist L, Rodriguez-Martinez H. « Assessment of sperm characteristics post-thaw and response to calcium ionophore in relation to fertility in Swedish dairy AI bulls. » Theriogenology 2000; 53:859-875).

Une autre partie du problème rencontré pour évaluer la fertilité d'un animal à partir de résultats de laboratoire résulte de problèmes sous-jacents à l'analyse en laboratoire elle-même. Pour être valide, un test en laboratoire devrait être objectif (créer peu d'erreur due au jugement de l'homme ou de biais), répétable (produire les même résultats lors de la répétition du test), précis (évaluer précisément un paramètre des spermatozoïdes), rapide et peu coûteux (Graham JK. « Assessment of sperm quality: a flow cytometric approach. » Anim Reprod Sci 2001; 68:239-247). Actuellement, peu de tests en laboratoire pour analyser la semence possèdent toutes ces caractéristiques.

Dans les dernières années, l'utilisation de la technique d'analyse CASA (acronyme anglais de Computer Assisted Sperm Analysis) et de la technique d'analyse de cytométrie en flux a permis une évolution des méthodes d'évaluation de qualité d'une semence en laboratoire.

Un inconvénient de ces techniques analyses multiparamétriques est de ne pas permettre une mesure in vitro prédictive de la fertilité in vivo. Cette absence de résultat probant peut être imputée aux paramètres et marqueurs utilisés pour ces tests qui présentent une trop faible corrélation avec le phénotype à prédire et/ou sont redondants et/ou sont limités en nombre.

Les approches haut débit regroupées sous le terme « omics » ou « phenomics » permettent des évaluations de plus en plus approfondies de la qualité d'une semence avec des perspectives intéressantes aussi bien en clinique humaine (Egea RR, Puchalt NG, Escrivá MM, Varghese AC. OMICS: « Current and future perspectives in reproductive medicine and technology. » J Hum Reprod Sci 2014; 7:73-92) qu'en agronomie (Robert C. « Challenges of functional genomics applied to farm animal gametes and pre-hatching embryos. » Theriogenology 2008; 70:1277-1287).

Un inconvénient pour l'utilisation en routine de ces techniques connues découle de leurs coûts.

Un autre inconvénient est la complexité de la mise en oeuvre de ces techniques.

Encore un inconvénient est que ces techniques connues sont réalisées à partir d'une fraction d'un échantillon et non sur un échantillon intact.

D'autres techniques ont été proposées pour permettre l'analyse de cellules intactes, et notamment pour déterminer leur lipidome (Jones JJ, Stump MJ, Fleming RC, Lay JO, Wilkins CL. « Strategies and data analysis techniques for lipid and phospholipid chemistry elucidation by intact cell MALDI-FTMS. » J Am Soc Mass Spectrom 2004; 15:1665-1674) ou leur protéome (Labas V, Spina L, Belleannee C, Teixeira-Gomes A-P, Gargaros A, Dacheux F, Dacheux J-L. « Analysis of epididymal sperm maturation by MALDI profiling and top-down mass spectrometry. » J Proteomics 2015; 113:226-243).

Un inconvénient de ces techniques d'analyse de cellules intactes est qu'elles ne permettent pas de caractériser des spermatozoïdes intacts pour un ensemble de caractères moléculaires et/ou structuraux de façon simultanée.

On connait également une technique d'analyse de la qualité d'une semence par spectroscopie infrarouge, qui consiste plus particulièrement à analyser un échantillon irradié sous un rayonnement dans l'infrarouge moyen (la longueur d'onde du rayonnement est comprise entre 2,5 $\mu$m et 25 $\mu$m), également connue sous l'acronyme de MIR (acronyme de l'anglais « mid-infrared»).

Lorsqu'un échantillon biologique est irradié par un rayonnement MIR, le rayonnement va être partiellement et sélectivement absorbé en fonction des liaisons chimiques des différentes molécules présentes dans l'échantillon. Un spectre infrarouge est donc composé de bandes d'absorption qui peuvent être attribuées à des groupements chimiques précis. La position des bandes dépend à la fois de la nature de la liaison, mais également de son environnement. Ainsi, la position de la bande d'absorption permet de relier ces bandes d'absorption à des molécules particulières comme les protéines, lipides ou glucides.

Des études ont été menées afin d'évaluer les performances de la technique de spectroscopie dans l'infrarouge moyen pour différentes applications. On peut par exemple citer une étude visant le typage bactérien (Helm D, Labischinski H, Schallehn G, Naumann D. « Classification and identification of bacteria by Fourier-transform infrared spectroscopy. » J Gen Microbiol 1991; 137:69-79; et Stamm I, Hailer M, Depner B, Kopp PA, Rau J. « Yersinia enterocolitica in diagnostic fecal samples from European dogs and cats: identification by fourier transform infrared spectroscopy and matrix-assisted laser desorption ionization-time of flight mass spectrometry. » J Clin Microbiol 2013; 51:887-893), une étude sur l'identification de pathologies telles que le cancer (Backhaus J, Mueller R, Formanski N, Szlama N, Meerpohl H-G, Eidt M, Bugert P. « Diagnosis of breast cancer with infrared spectroscopy from sérum samples. » Vib Spectrosc 2010; 52:173-177; et Kallenbach-Thieltges A, Großerüschkamp F, Mosig A, Diem M, Tannapfel A, Gerwert K. « Immunohistochemistry, histopathology and infrared spectral histopathology of colon cancer tissue sections. » J Biophotonics 2013; 6:88-100; et Lewis PD, Lewis KE, Ghosal R, Bayliss S, Lloyd AJ, Wills J, Godfrey R, Kloer P, Mur LAJ. « Evaluation of FTIR spectroscopy as a diagnostic tool for lung cancer using sputum. » BMC Cancer 2010; 10:640), ou des pathologies articulaires (Canvin JMG, Bernatsky S, Hitchon CA, Jackson M, Sowa MG, Mansfield JR, Eysel HH, Mantsch HH, El-Gabalawy HS. « Infrared spectroscopy: shedding light on synovitis in patients with rheumatoid arthritis. » Rheumatol Oxf Engl 2003; 42:76-82), ou encore la détection de contaminants dans l'industrie alimentaire (de Carvalho BMA, de Carvalho LM, Reis Coimbra JS dos, Minim LA, de Souza Barcellos E, da Silva Júnior WF, Detmann E, de Carvalho GGP. « Rapid détection of whey in milk powder samples by spectrophotometric and multivariate calibration. » Food Chem 2015; 174:1-7).

L'analyse spectroscopique infrarouge par ondes évanescentes connue sous l'acronyme FEWS (de l'anglais « Fiber Evanescent Wave Spectroscopy ») permet de travailler dans le MIR. Cette analyse est basée sur l'utilisation d'un capteur composé d'une fibre optique en verre de chalcogénure qui est décrite, par exemple, dans les documents FR2958403, WO2013017324, et FR1450661. Lorsqu'une onde lumineuse se propage dans une fibre optique, elle le fait par réflexions multiples, l'ensemble de ces rayons constituant l'onde évanescente. Cette onde peut alors être absorbée par un milieu en contact avec la fibre.

Cette technique offre de nombreux avantages. Contrairement aux analyses discrètes qui visent à rechercher et/ou quantifier certains métabolites définis a priori, l'analyse MIR permet d'obtenir une image globale du profil métabolique de l'échantillon en une seule analyse prenant ainsi en compte les interactions entre molécules. La particularité du capteur à fibre optique en fait un outil pouvant être utilisé en milieu liquide tout en étant peu sensible à la teneur en eau des échantillons contrairement aux autres techniques d'acquisition. Le résultat est rapide et ne nécessite pas de traitement particulier des échantillons.

Plusieurs utilisations de la spectroscopie Raman ont déjà montré des aperçus du potentiel de cette technique appliquée au domaine de la reproduction (Mallidis C, Sanchez V, Wistuba J, Wuebbeling F, Burger M, Fallnich C, Schlatt S. « Raman microspectroscopy: shining a new light on reproductive medicine. » Hum Reprod Update 2014; 20:403-414). Cette technique a été appliquée à l'évaluation de l'intégrité de l'ADN des spermatozoïdes (Mallidis C, Wistuba J, Bleisteiner B, Damm OS, Gross P, Wübbeling F, Fallnich C, Burger M, Schlatt S. « In situ visualization of damaged DNA in human sperm by Raman microspectroscopy. » Hum Reprod Oxf Engl 2011; 26: 1641-1649; et Mallidis C, Schlatt S, Wistuba J, Fallnich C, Gross P, Burger M, Wuebbeling F. « Means and methods for assessing sperm nuclear DNA structure. » WO2013064159 A1, 2013), à l'analyse de l'acrosome pour déterminer le pouvoir fécondant d'un spermatozoïde (李铮, 朱勇, 刘锋, 何琳, 刘宇飞. « Sperm acrosome zone Raman spectrum peak and use thereof. » CN103698310 A, 2014) ou à l'analyse du plasma séminal à des fins de diagnostic (Huang Z, Chen X, Chen Y, Chen J, Dou M, Feng S, Zeng H, Chen R. « Raman spectroscopic characterization and differentiation of seminal plasma. » J Biomed Opt 2011; 16: 110501-1105013).

Elle n'a cependant jamais été utilisée jusqu'à présent pour caractériser un échantillon complet de semence.

L'article: PEREZ-MARIN C C ET AL: «Assessment of porcine semen concentration by near InfraRed Spectroscopy (NIRS)»,REPRODUCTION IN DOMESTIC ANIMALS, vol. 42, no. Supplément 2, 1 septembre 2007 (2007-09-01), p. 92, divulgue un procédé selon le préambule de la revendication 1.

## 3. Objectifs de l'invention

L'invention a donc notamment pour objectif de pallier les inconvénients de l'état de la technique cités ci-dessus.

Plus précisément, l'invention a pour objectif de fournir un procédé de détermination de la qualité d'une semence d'un animal vertébré qui soit efficace et fiable pour tout type de semence.

Un autre objectif de l'invention est de fournir une telle technique qui soit simple et rapide à mettre en œuvre.

L'invention a également pour objectif de proposer un procédé de détermination de la qualité d'une semence d'un animal vertébré qui soit d'un coût de revient réduit.

## 4. Exposé de l'invention

Ces objectifs, ainsi que d'autres qui apparaîtront par la suite sont atteints à l'aide d'un procédé de détermination de la qualité d'une semence d'un animal vertébré.

Dans le cadre de l'invention, on entend le terme "animal" dans son acception courante, à savoir un être vivant hétérotrophe non humain. En d'autres termes, l'invention concerne un procédé de détermination de la qualité d'une semence d'un vertébré non humain. Il peut par exemple s'agir de gros bétail, de petit bétail, d'un aviaire ou d'un poisson. L'invention est définie par le procédé de la revendication 1.

Ce procédé de détermination de la qualité d'une semence d'un animal vertébré comprend les étapes suivantes :

- mesure d'au moins un spectre d'absorption d'un échantillon de ladite semence ;
- sélection d'un nombre n, avec n ≥ 4, de nombres d'ondes $\sigma_{j\,(j\in[1;n])}$ caractéristiques des semences de la race ou de l'espèce dudit animal ;
- détermination à partir dudit ou desdits spectres d'absorption d'une valeur de l'absorption $X_j$ et/ou d'une valeur de la dérivée seconde de l'absorption $X_j"\,_{(j\in[1;n])}$ pour chacun desdits n nombres d'ondes $\sigma_{j\,(j\in[1;n])}$ ;
- calcul d'un taux de non-retour Y à un nombre prédéfini de jours à partir desdites valeurs de l'absorption $X_j$ et/ou de la dérivée seconde de l'absorption $X_j"$ précédemment déterminées.

Ainsi, de façon inédite, l'invention propose d'utiliser le spectre d'absorption d'une semence pour évaluer la qualité d'une semence d'un animal, à partir d'un nombre réduit de nombres d'ondes représentatifs de variables explicatives caractéristiques des semences de la race ou de l'espèce de l'animal.

L'invention permet notamment de déterminer un taux de non-retour, par exemple un taux de non retour en chaleur à 22, 28, 30, 56, 90 ou 120 jours, pour 98% des semences d'une race ou d'une espèce animale avec une précision de ± 10 points, à partir de seulement 20 variables explicatives, et avec une précision de ± 5 points pour 86 % des semences. On rappelle que le taux de non-retour à J jours (TNRJ) est une estimation du résultat de l'insémination, succès ou échec, basée sur l'absence d'un retour en oestrus enregistré au cours d'un intervalle de J jours après l'acte d'insémination. Après J jours, les vaches n'étant pas de retour en oestrus sont considérées comme gestantes.

Il convient par ailleurs de noter que l'invention permet d'évaluer la qualité des semences de toutes les races ou les espèces d'animaux vertébrés, sans exception, de façon rapide et à moindre coût.

Dans le cadre de l'invention, on entend par race animale, au sein d'une même espèce animale, une population d'individus homozygotes pour un certain nombre de caractères conditionnant un ensemble de traits ou particularités morphologiques et une même tendance générale d'aptitudes au sein d'une même espèce animale, telle que par exemple une race bovine, une race chevaline, une race porcine, une race ovine, une race caprine, une race de canard, une race de poule, une race d'oie, une race de dindon, une race de lapin,

Selon un aspect particulier de l'invention, ledit taux de non-retour Y est calculé selon la loi mathématique

$$Y = \beta_0 + \sum_{j=1}^{..} \beta_j X_j{}''$$

où $X_j{}''_{(j\in[1;n])}$ est la dérivée seconde normalisée de l'absorption pour le nombre d'ondes $\sigma_j$ et les coefficients de pondération $\beta_0$ et $\beta_{j\ (j\in[1;n])}$ sont des constantes.

Le taux de non retour est ainsi calculé de façon déterministe.

Dans le procédé de l'invention, les valeurs desdits coefficients de pondération sont obtenues à partir d'un traitement des mesures des spectres d'absorption d'une pluralité d'échantillons de semence d'une population de vertébré de référence, dont les taux de non-retour sont connus.

Selon un mode de réalisation avantageux de l'invention, lors de ladite étape de mesure, au moins 2, de préférence au moins 3 spectres d'absorption d'un échantillon de ladite semence sont mesurés et ladite étape de détermination de valeurs de l'absorption et/ou de dérivées secondes de l'absorption comprend une étape de réalisation d'une moyenne desdits spectres mesurés à partir de laquelle sont déterminés lesdites valeurs d'absorption et/ou des dérivées secondes de l'absorption.

On réduit ainsi la sensibilité aux artéfacts de mesure et aux perturbations.

De façon avantageuse, le nombre n de nombres d'ondes $\sigma_{j\ (j\in[1;n])}$ est supérieur ou égal à 7, de préférence supérieur ou égal à 9, encore plus préférentiellement supérieur ou égal à 13, encore encore plus préférentiellement supérieur ou égal à 20.

On augmente la précision de l'évaluation de la qualité de la semence en prenant en compte un nombre plus important, mais restant limité, de nombres d'ondes.

L'invention ne se limite pas à des nombres d'ondes supérieurs ou égaux à 4. Le nombre n de nombres d'ondes $\sigma_{j\ (j\in[1;n])}$ peut être égal à 2 ou 3 sans sortir du cadre de l'invention.

Selon un aspect préféré de l'invention, lesdits nombres d'ondes sont représentatifs chacun d'une molécule ou d'un ensemble de molécules choisies dans le groupe comprenant au moins :

- lipides ;
- glucides ;
- protéines ;
- acides nucléiques ;
- combinaison d'une molécule de lipide, de glucide, de protéine, ou d'acide nucléique avec au moins une autre molécule de lipide, de glucide, de protéine, ou d'acide nucléique.

Par exemple, pour un taureau, on peut ainsi choisir parmi environ 600 variables explicatives.

Dans un mode de réalisation particulier de l'invention, ledit vertébré est un taureau et le taux de non-retour est un taux de non-retour à 90 jours, ledit taureau étant issu d'une race sélectionnée dans le groupe comprenant au moins l'Abondance, la Béarnaise, la Bordelaise, la Bretonne pie noir, la Brune, la Froment du Léon, la Jersiaise, la Montbéliarde, la Pie rouge des plaines, la Prim'Holstein, la Rouge flamande, la Bleue du nord, la Normande, la Salers, la Tarentaise.

De préférence, au moins un desdits nombres d'ondes $\sigma_{j\ (j\in[1;n])}$ est choisi dans la plage de nombre d'ondes [960 cm$^{-1}$ ; 1100 cm$^{-1}$], [1440 cm$^{-1}$ ; 1550 cm$^{-1}$] ou [2800 cm$^{-1}$ ; 3200 cm$^{-1}$] ou dans la bande amide B.

Selon un aspect particulier de l'invention, lesdits nombres d'ondes $\sigma_{j\ (j\in[1;n])}$ sont choisis dans le groupe comprenant au moins 955,0 ±0,1 cm$^{-1}$, 963,1 ±0,1 cm$^{-1}$, 1012,1 ±0,1 cm$^{-1}$ , 1036,6 ±0,1 cm$^{-1}$, 1095,8 ±0,1 cm$^{-1}$, 1124,3 ±0,1 cm$^{-1}$, 1136,6 ±0,1 cm$^{-1}$ ,1365,1 ±0,1 cm$^{-1}$, 1383,5 ±0,1 cm$^{-1}$, 1428,4 ±0,1 cm$^{-1}$, 1444,7 ±0,1 cm$^{-1}$, 1452,9 ±0,1 cm-1, 1503,9 ±0,1 cm$^{-1}$, 1520,2 ±0,1 cm$^{-1}$, 2805,7 ±0,1 cm$^{-1}$, 2956,7 ±0,1 cm$^{-1}$ , 2969,0 ±0,1 cm$^{-1}$, 2987,4 ±0,1 cm$^{-1}$, 3089,4 ±0,1 cm$^{-1}$, 3091,4 ±0,1 cm$^{-1}$ .

Dans un mode de réalisation particulier de l'invention, lesdits nombres d'ondes $\sigma_{j\ (j\in[1;n])}$ sont choisis dans le groupe comprenant au moins 3165 ± 1 cm$^{-1}$, 3136 ± 1 cm$^{-1}$, 3103 ± 1 cm$^{-1}$, 3071 ± 1 cm$^{-1}$, 3026 ± 1 cm$^{-1}$, 2977 ± 1 cm$^{-1}$, 2975 ± 1 cm$^{-1}$, 1736 ± 1 cm$^{-1}$, 1689 ± 1 cm$^{-1}$, 1661 ± 1 cm$^{-1}$, 1618 ± 1 cm$^{-1}$, 1514 ± 1 cm$^{-1}$, 1448 ± 1 cm$^{-1}$, 1430 ± 1 cm$^{-1}$, 1344 ± 1 cm$^{-1}$, 1338 ± 1 cm$^{-1}$, 1336 ± 1 cm$^{-1}$, 1316 ± 1 cm$^{-1}$, 1214 ± 1 cm$^{-1}$, 1183 ± 1 cm$^{-1}$, 1103 ± 1 cm$^{-1}$, 1099 ± 1 cm$^{-1}$, 1020 ± 1 cm$^{-1}$, 975 ± 1 cm$^{-1}$.

Dans un mode de réalisation particulier de l'invention, le nombre n desdits nombres d'ondes $\sigma_{j\ (j\in[1;n])}$ sélectionnés est égal à treize et en ce que les valeurs desdits treize nombres d'ondes $\sigma_{j\ (j\in[1;n])}$ sélectionnés sont : 3165 ±

1 cm$^{-1}$, 3136 ± 1 cm$^{-1}$, 3103 ± 1 cm$^{-1}$, 2975 ± 1 cm$^{-1}$, 1736 ± 1 cm$^{-1}$, 1661 ± 1 cm$^{-1}$, 1515 ± 1 cm$^{-1}$, 1448 ± 1 cm$^{-1}$, 1430 ± 1 cm$^{-1}$, 1316 ± 1 cm$^{-1}$, 1214 ± 1 cm$^{-1}$, 1020 ± 1 cm$^{-1}$, 975 ± 1 cm$^{-1}$.

Dans un mode de réalisation particulier de l'invention, le nombre n desdits nombres d'ondes $\sigma_{j\,(j\in[1;n])}$ sélectionnés est égal à quinze et en ce que les valeurs desdits quinze nombres d'ondes $\sigma_{j\,(j\in[1;n])}$ sélectionnés sont : 3071 ± 1 cm$^{-1}$, 3026 ± 1 cm$^{-1}$, 2977 ± 1 cm$^{-1}$, 1689 ± 1 cm$^{-1}$, 1618 ± 1 cm$^{-1}$, 1515 ± 1 cm$^{-1}$, 1430 ± 1 cm$^{-1}$, 1344 ± 1 cm$^{-1}$, 1338 ± 1 cm$^{-1}$, 1336 ± 1 cm$^{-1}$, 1183 ± 1 cm$^{-1}$, 1103 ± 1 cm$^{-1}$, 1099 ± 1 cm$^{-1}$, 1020 ± 1 cm$^{-1}$, 975 ± 1 cm$^{-1}$.

De préférence, ladite étape de mesure d'au moins un spectre d'absorption comprend une étape de préparation dudit échantillon à partir de ladite semence.

Dans un mode de réalisation particulier de l'invention, le procédé de détermination de la qualité d'une semence tel que décrit ci-dessus comprend une étape de comparaison dudit taux de non-retour Y avec un seuil prédéterminé, permettant de sélectionner ladite semence pour des besoins de reproduction dans le cas où ledit taux de non-retour Y est supérieur ou égal audit seuil prédéterminé.

On dispose ainsi d'une technique efficace et particulièrement simple à mettre en oeuvre pour sélectionner des semences de qualité.

Dans un mode de réalisation avantageux de l'invention, ledit seuil est supérieur ou égal à 0,4, de préférence supérieur ou égal à 0,5, encore plus préférentiellement supérieur ou égal à 0,6.

## 5. Liste des figures

D'autres caractéristiques et avantages de l'invention apparaîtront plus clairement à la lecture de la description suivante d'un mode de réalisation de l'invention, donné à titre de simple exemple illustratif et non limitatif, et des dessins annexés parmi lesquels :

- la figure 1 illustre, sous forme de schéma-bloc, les étapes d'un exemple de mode de réalisation d'un procédé de détermination de la qualité d'une semence d'un taureau selon l'invention ;

- la figure 2 représente des spectres MIR, dérivés et normalisés acquis à partir d'une paillette, d'un surnageant et d'un culot ;

- la figure 3 est une représentation de la variance d'une paillette et d'un culot provenant d'un même éjaculat en fonction du nombre d'onde ;

- les figures 4A, 4B et 5 illustrent la corrélation entre des valeurs de TNR90 calculée par un modèle à 20 nombres d'ondes pour des échantillons d'éjaculat de taureaux Prim'holstein et des valeurs connues pour ces mêmes échantillons, respectivement pour 70 échantillons, pour 16 autres échantillons et pour l'ensemble de ces 86 échantillons ;

- la figure 6 est une autre représentation de la figure 5 sur laquelle on a ajouté deux droites représentatives d'un écart sur la valeur du TNR90 par rapport au modèle respectivement de +5 points et de -5 points.

## 6. Description détaillée de l'invention

### 6.1. Protocole expérimental

Les échantillons analysés sont des éjaculats bovins sous la forme de paillettes, conservées dans l'azote liquide. Les analyses préliminaires ont été réalisées sur 130 éjaculats provenant de 50 taureaux différents de race Prim'Holstein. L'indicateur TNR90brut a été utilisé pour qualifier la qualité des éjaculats.

### 6.1.1. Préparation des échantillons

Lors de la phase de préparation des échantillons, dans une première étape, les paillettes sont décongelées au bain-marie à 37 °C pendant 30 secondes. Dans une deuxième étape, les paillettes sont analysées. Pour cela, le contenu des paillettes est placé dans un tube Eppendorf 1,5 ml. Sept microlitres sont alors déposés sur le capteur pour l'acquisition spectrale MIR d'une « paillette ». Dans une troisième étape un surnageant est extrait par une centrifugation à 3500 g pendant 5 min à 15 °C. Le surnageant est alors déposé sur le capteur pour l'acquisition du spectre « surnageant ». Dans une quatrième étape, le culot est rincé avec 600 $\mu$l de NaCl 0,9 % puis une centrifugation est faite. Le culot est de nouveau suspendu dans 3,5 $\mu$l de NaCl 0,9 % et déposé sur le capteur pour l'acquisition du spectre « culot ».

### 6.1.2. Acquisition spectrale MIR d'une paillette

Les spectres sont acquis en absorbance de 4000 à 400 $cm^{-1}$. La résolution spectrale est fixée à 4 $cm^{-1}$, avec un facteur de « zero-filling » de 2, et 64 numérisations sont enregistrés.

Un capteur est placé dans le spectromètre, la ligne de base est enregistrée pour étalonner l'appareil, puis 7 $\mu$l d'échantillon sont déposés sur le capteur. Le spectre est enregistré au bout de 6 minutes.

### 6.1.3. Traitement des spectres

Les spectres sont analysés dans le domaine 3800-940$cm^{-1}$, domaine d'absorption de la majorité des biomolécules. Une ligne droite est générée de 2800 à 1800 $cm^{-1}$ pour éliminer la contribution du $CO_2$, puis la dérivée seconde (algorithme de Savisky-Golay avec 13 points de lissage) est calculée de 3200 à 2800$cm^{-1}$ et de 1800 à 940 $cm^{-1}$. Ensuite, une normalisation vectorielle des dérivées secondes est réalisée. Des critères de qualité sont définis pour rejeter les spectres non conformes.

### 6.1.4. Choix de la matrice pour la prédiction de la qualité de la semence

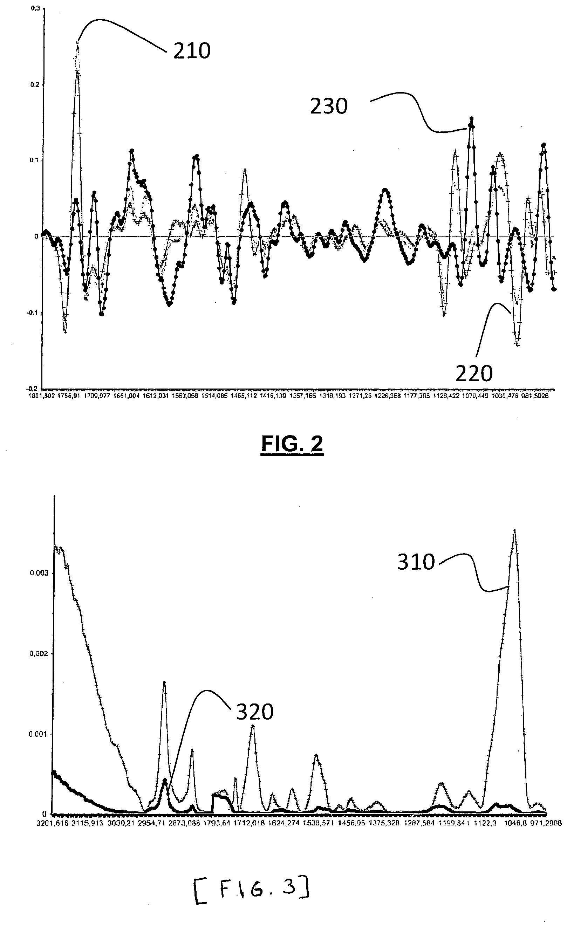

Trois types d'acquisitions ont été réalisées et comparées : éjaculat ou semence totale, culot et surnageant (centrifugation de la semence). Une observation des spectres (voir la **figure 2)** met en évidence que les spectres « paillettes » 210 et « surnageant » 220 présentent de nombreuses similarités. On réalise une Analyse en Composantes Principales (ACP) sur les données afin de comparer les spectres. Il apparaît sur la carte factorielle que les spectres acquis à partir du surnageant sont très proches de ceux acquis à partir de l'éjaculat totale. Ainsi les spectres acquis à partir des paillettes contiennent une information biochimique très proche de celle du liquide séminal. Or, ce dernier est fortement dilué (de 3 à 30 fois) dans un tampon avant la congélation et n'est donc pas déterminant de la qualité spécifique de la semence diluée/congelée/décongelée du taureau puisque tous les éjaculats sont traités de la même manière. Du fait de la dilution plus ou moins importante de l'éjaculat, les spectres MIR des paillettes reflètent nécessairement la composition biochimique du milieu de dilution. Cette contribution aux spectres MIR risque donc de masquer celle des spermatozoïdes qui sont censés contenir l'information spectrale qui établit la différence du point de vue de la fertilité. Par ailleurs, si un défaut de fertilité lié seulement à une moindre qualité du liquide séminal (fructose, pH,...) est considéré, il est attendu que ce défaut soit compensé par la dilution dans le milieu tampon. Il s'avère donc que l'information spectrale qui établit la différence soit à rechercher sur les cellules seules, plutôt que sur l'échantillon global plus ou moins dilué. La variabilité des mesures entre paillette et culot 230 a également été prise en compte. Pour cela les variances ont été calculées sur les 3 spectres acquis pour un même éjaculat. Les spectres bruts, c'est-à-dire les spectres non dérivés, ont tout d'abord été normalisés par la méthode d'anti-éparpillement MSC (acronyme anglais de Multiplicative Scatter Correction) afin de s'affranchir notamment des variations de ligne de base. Comme le montre la **figure 3,** avec une représentation de la variance en fonction du nombre d'ondes (en $cm^{-1}$) pour une analyse des paillettes 310 et du culot 320, il apparaît de manière générale que les signaux acquis à partir de la semence totale présentent plus de variabilité notamment dans le domaine 1000-940$cm^{-1}$.

Les mesures sont donc réalisées sur le culot de centrifugation contenant essentiellement les spermatozoïdes.

### 6.2. Construction de modèles de détermination de la qualité d'une semence

### 6.2.1. Échantillons de référence

Quatre-vingt-six éjaculats, provenant de 40 taureaux de race Prim'Holstein, dont le taux de non-retour à 90 jours brut, ou TNR90brut, est connu, sont utilisés pour l'établissement de la loi ou équation de détermination de la qualité de l'éjaculat.

Ces éjaculats proviennent de taureaux âgés de 11 mois à 10,5 ans au moment de la collecte de leurs éjaculats. La répartition du nombre d'éjaculat(s) par taureau est la suivante : 8, 18 et 14 taureaux ont produit respectivement 1, 2 ou 3 éjaculats.

### 6.2.2. Préparation des paillettes

Pour chaque éjaculat, six paillettes sont décongelées en plaçant ces dernières au bain-marie à 37 °C pendant 30 secondes.

Le contenu des six paillettes est vidé dans un tube Eppendorf 1,5 ml, puis il subit une centrifugation à 3500 g pendant 5 min à 15 °C. Le surnageant est éliminé et le culot est rincé avec 600 $\mu$l de NaCl 0,9 %. Cette étape est renouvelée une fois. À la suite du second rinçage, une nouvelle centrifugation est appliquée et le culot est de nouveau

suspendu dans 10 $\mu$l de NaCl 0,9 %.

### 6.2.3. Acquisition des spectres

A partir de cette préparation, on procède à l'acquisition de trois spectres pour chaque éjaculat. L'acquisition de chacun des spectres est réalisée avec un pas de 2 cm$^{-1}$, sur un spectromètre dont la précision, quelle que soit la position, c'est-à-dire le nombre d'onde, est de 0,1 cm$^{-1}$.

Pour acquérir chacun des spectres, 7 $\mu$l de la suspension formée du culot et de la solution saline sont déposés dans le capteur. Les spermatozoïdes sont soumis à un rayonnement dans le moyen infrarouge et leur spectre d'absorption est enregistré au bout de 6 minutes.

### 6.2.4. Analyse des données

Les spectres sont soumis à un contrôle qualité portant sur différents critères avant d'être sélectionnés pour la suite des analyses. Les critères pris en compte pour le contrôle qualité comprennent l'intensité du signal, les interférences, le rapport signal/bruit et la teneur en eau.

La moyenne des trois spectres acquis est effectuée pour obtenir un spectre moyenné de l'éjaculat.

Le spectre moyenné est traité selon la procédure décrite précédemment au paragraphe 6.1.3.

Pour l'ensemble des échantillons, les spectres sont répartis en deux catégories, les spectres de calibration provenant de l'analyse de 70 éjaculats et les spectres de validation provenant de l'analyse des 16 éjaculats restants. Les spectres de calibration servent à construire le modèle visant à mettre en relation une variable à expliquer, ici le TNR90brut, et des variables explicatives c'est-à-dire une sélection optimisée de nombres d'ondes du spectre. Une fois le modèle optimisé, les spectres de validation servent à évaluer la performance prédictive dudit modèle.

La réduction de variables explicatives est réalisée par un algorithme génétique associé à une R-PLS avec validation croisée de 10 %. Une fois cette réduction réalisée, le choix des variables explicatives est optimisé par la répétition de 100 régressions linéaires (RL), avec une validation croisée portant sur 10 % de la population initiale. Une validation des variables explicatives retenues est effectuée en les intégrant dans la loi ou équation linéaire servant à prédire les échantillons « inconnus ».

### 6.2.5. Construction de modèles de détermination de la qualité d'une semence

Les échantillons de référence sont scindés en une sous-population de calibration et une sous-population de validation. L'apprentissage est réalisé sur 4/5ème des éjaculats, la validation sur le 5ème restant et pour chacune des sous-populations, on maximise le nombre d'éjaculats provenant de taureaux différents tout en ayant une représentativité proportionnelle des individus dans 3 classes de TNR90 définies comme suit : TNR90 <40 %, 40 %$\leq$TNR90$\leq$ 50 % et TNR90>50 %.

#### 6.2.5.1. Modèle mathématique

Le modèle mathématique utilisé est défini par la formule $Y = \beta_0 + \sum_{j=1}^{n} \beta_j X_j''$ , dans laquelle :

Y est le TNR90 calculé pour l'éjaculat ;
n (n$\geq$4) est le nombre de nombres d'ondes $\sigma_j$ considérés dans le modèle ;
$X_j''$ est la dérivée seconde normalisée de la valeur de l'absorption pour le nombre d'ondes $\sigma_j$ ;
$\beta_0$ est le décalage à l'origine ;
$\beta_j$ (1$\leq$j$\leq$n) est le coefficient de pondération de la valeur de la dérivée seconde normalisée de l'absorption $X_j''$, borné par son erreur standard.

#### 6.2.5.2. Exemples de modèles

Cinq modèles sont construit à partir respectivement de 7, 9, 13, 15 et 20 nombres d'ondes, en minimisant l'erreur de prédiction, RMSEP (acronyme en anglais de « Root-Mean-Square Error of Prédiction », prédiction selon l'erreur moyenne quadratique).

Les domaines spectraux des nombres d'ondes choisis concernent notamment le domaine d'absorption des lipides 3200-2800 cm$^{-1}$, des protéines (bande amide B et domaine 1440-1500 cm$^{-1}$) ainsi que de l'ADN (1514 cm$^{-1}$, 1099 cm$^{-1}$ et 975 cm$^{-1}$).

a) *Modèle à 7 nombres d'ondes*

Ce modèle est détaillé dans le tableau 1, ci-dessous. il présente un coefficient de détermination calculé $R^2$ (ou « Multiple R-Squared » en anglais) de 0,4804 et un RMSEP de 4,8%.

| Variable explicative j | Nombre d'onde $\sigma$ (cm-1) | Coefficient $\beta j$ | Erreur Standard | Résultat de test | Probabilité |
|---|---|---|---|---|---|
| | | Estimate | Std. Error | t value | Pr (>\|t\|) |
| 0 | | 0.2277 | 0.2503 | 0.910 | 0.365812 |
| 1 | 2956.7 | 9.3246 | 2.4387 | 3.824 | 0.000263 |
| 2 | 1428.4 | -10.4157 | 4.4484 | -2.341 | 0.021764 |
| 3 | 1383.5 | -15.7746 | 5.2130 | -3.026 | 0.003356 |
| 4 | 1365.1 | 19.7086 | 5.7690 | 3.416 | 0.001011 |
| 5 | 1095.8 | -6.8730 | 1.4857 | -4.626 | 1.46e-05 |
| 6 | 1036.6 | 7.7556 | 2.3304 | 3.328 | 0.001337 |
| 7 | 963.1 | 3.7342 | 1.1538 | 3.236 | 0.001777 |

*Tableau 1*

| Distribution des résidus en minimum, maximum et quartiles : | | | | |
|---|---|---|---|---|
| Minimum | 1er quartile | Médian | 3e quartile | Maximum |
| -0,144853 | -0,028759 | 0,000373 | 0,033174 | 0,109250 |

Écart-type résiduel (« Residual standard error » en anglais): 0,05298 avec 78 degrés de liberté
Coefficient de détermination ajusté ($R^2$ ajusté ou « Adjusted R-Squared » en anglais) : 0,4337
F-statistique (F-value ou valeur F du test de Fisher) : 10,3 sur 78 degrés de liberté, P-value (valeur P du test de Fisher) : 4,455e-09

b) Modèle à 9 nombres d'ondes

Ce modèle à 9 nombres d'ondes est détaillé dans le tableau 2, ci-dessous. il présente un coefficient de détermination calculé $R^2$ de 0,5884 et un RMSEP de 4,49%.

| Variable explicative j | Nombre d'onde $\sigma$ (cm-1) | Coefficient $\beta$ | Erreur Standard | Résultat de test | Probabilité |
|---|---|---|---|---|---|
| | | Estimate | Std. Error | t value | Pr (>\|t\|) |
| 0 | | -0.06038 | 0.30929 | -0.195 | 0.84573 |
| 1 | 2956.7 | 13.30697 | 2.44983 | 5.432 | 6.46e-07 |
| 2 | 1503.9 | -7.95636 | 2.95590 | -2.692 | 0.00874 |
| 3 | 1444.7 | 7.51796 | 3.27999 | 2.292 | 0.02467 |
| 4 | 1428.4 | -5.34138 | 4.70264 | -1.136 | 0.25960 |
| 5 | 1383.5 | -11.74606 | 4.99481 | -2.352 | 0.02128 |
| 6 | 1365.1 | 22.37749 | 5.23880 | 4.271 | 5.56e-05 |
| 7 | 1095.8 | -8.07636 | 1.48922 | -5.423 | 6.68e-07 |
| 8 | 1036.6 | 9.59107 | 2.14903 | 4.463 | 2.76e-05 |
| 9 | 963.1 | 3.14150 | 1.14317 | 2.748 | 0.00748 |

*Tableau 2*

| Distribution des résidus en minimum, maximum et quartiles : | | | | |
|---|---|---|---|---|
| Minimum | 1er quartile | Médian | 3e quartile | Maximum |
| -0,115369 | -0,023260 | -0,005633 | 0,021377 | 0,122507 |

Écart-type résiduel : 0,04777 avec 76 degrés de liberté
Coefficient de détermination ajusté : 0,5397
F-statistique : 12,07 sur 76 degrés de liberté, P-value : 1,325e-11

c) *Modèle à 13 nombres d'ondes*

Ce modèle est détaillé dans le tableau 3, ci-dessous. Il présente un coefficient de détermination calculé R2 (ou « Multiple R-Squared » en anglais) de 0,2972 et un RMSEP de 6,3%.

| Variable explicative j | Nombre d'onde σ (cm⁻¹) | Coefficient β | Erreur Standard | Résultat de test | Probabilité |
|---|---|---|---|---|---|
| | | Estimate | Std. Error | t value | Pr (>\|t\|) |
| 0 | | -0.1649 | 0.2345 | -0.703 | 0.48275 |
| 1 | 3164.886 | -1.2917 | 3.4928 | -0.370 | 0.71191 |
| 2 | 3136.318 | 3.9653 | 4.0591 | 0.977 | 0.32979 |
| 3 | 3103.67 | -1.8260 | 4.33 | -0.422 | 0.67368 |
| 4 | 2975.115 | -6.4489 | 3.4555 | -1.866 | 0.06346 |
| 5 | 1736.504 | 3.5545 | 1.5065 | 2.359 | 0.01926 |
| 6 | 1661.004 | 0.244 | 1.339 | 0.182 | 0.85558 |
| 7 | 1514.085 | -13.7841 | 2.3808 | -5.790 | 2.68e-08 |
| 8 | 1448.787 | 5.1977 | 1.584 | 3.281 | 0.00122 |
| 9 | 1430.422 | -23.5023 | 3.9896 | -5.891 | 1.59e-08 |
| 10 | 1316.152 | 6.6227 | 3.62 | 1.829 | 0.06881 |
| 11 | 1214.124 | 6.0439 | 3.0219 | 2 | 0.04684 |
| 12 | 1020.273 | -10.1756 | 2.5485 | -3.993 | 9.16e-05 |
| 13 | 975.3806 | -6.2081 | 1.4327 | -4.333 | 2.32e-05 |

*Tableau 3*

| Distribution des résidus en minimum, maximum et quartiles : | | | | |
|---|---|---|---|---|
| Minimum | 1er quartile | Médian | 3e quartile | Maximum |
| -0.193993 | -0.033218 | 0.001113 | 0.039355 | 0.189818 |

Écart-type résiduel (« Residual standard error » en anglais): 0,06529 avec 201 degrés de liberté
Coefficient de détermination ajusté (R2 ajusté ou « Adjusted R-Squared » en anglais) : 0,2517
F-statistique (F-value ou valeur F du test de Fisher) : 6,537 sur 13 et 201 degrés de liberté, P-value (valeur P du test de Fisher) : 2,492e-10

d) *Modèle à 15 nombres d'ondes*

Ce modèle est détaillé dans le tableau 4, ci-dessous. Il présente un coefficient de détermination calculé R2 (ou « Multiple R-Squared » en anglais) de 0,3861 et un RMSEP de 5,9%.

| Variable explicative j | Nombre d'onde $\sigma$ (cm$^{-1}$) | Coefficient $\beta$ | Erreur Standard | Résultat de test | Probabilité |
|---|---|---|---|---|---|
| | | Estimate | Std. Error | t value | Pr (>\|t\|) |
| 0 | | 0.1618 | 0.2033 | 0.796 | 0.427213 |
| 1 | 3071.021 | 12.198 | 4.3248 | 2.82 | 0.005281 |
| 2 | 3026.129 | 3.2041 | 3.8144 | 0.84 | 0.401918 |
| 3 | 2977.156 | -6.9854 | 3.4328 | -2.035 | 0.043185 |
| 4 | 1689.572 | 1.1852 | 0.7567 | 1.566 | 0.11887 |
| 5 | 1618.152 | -4.0715 | 1.5196 | -2.679 | 0.007995 |
| 6 | 1514.085 | -8.8803 | 2.4751 | -3.588 | 0.00042 |
| 7 | 1430.422 | -21.7955 | 3.7797 | -5.767 | 3.05e-08 |
| 8 | 1344.719 | -18.6905 | 6.149 | -3.040 | 0.002687 |
| 9 | 1338.598 | 38.3873 | 14.814 | 2.591 | 0.01027 |
| 10 | 1336.557 | -29.9668 | 13.9565 | -2.147 | 0.032991 |
| 11 | 1183.516 | 9.7122 | 3.5233 | 2.757 | 0.006384 |
| 12 | 1103.935 | 9.9713 | 2.9172 | 3.418 | 0.000765 |
| 13 | 1099.854 | -9.4505 | 2.0387 | -4.636 | 6.43e-06 |
| 14 | 1020.273 | -8.8229 | 2.4303 | -3.630 | 0.00036 |
| 15 | 975.3806 | -2.7333 | 1.3692 | -1.996 | 0.047276 |

*Tableau 4*

| Distribution des résidus, en minimum, maximum et quartiles : | | | | |
|---|---|---|---|---|
| Minimum | 1$^{er}$ quartile | Médian | 3$^e$ quartile | Maximum |
| -0,178825 | -0,031587 | 0,001598 | 0,036951 | 0,163905 |

Écart-type résiduel (« Residual standard error » en anglais): 0,06133 avec 199 degrés de liberté

Coefficient de détermination ajusté (R$^2$ ajusté ou « Adjusted R-Squared » en anglais) : 0,3398

F-statistique (F-value ou valeur F du test de Fisher) : 8,343 sur 15 et 199 degrés de liberté, P-value (valeur P du test de Fisher) : 1,246e-14

e) <u>Modèle à 20 nombres d'ondes</u>

Ce modèle à 20 nombres d'ondes est détaillé dans le tableau 5, ci-dessous. il présente un coefficient de détermination calculé R$^2$ de 0,7785 et un RMSEP de 3,29%.

| Variable explicative j | Nombre d'onde $\sigma$ (cm-1) | Coefficient $\beta$ | Erreur Standard | Résultat de test | Probabilité |
|---|---|---|---|---|---|
| | | Estimate | Std. Error | t value | Pr (>\|t\|) |
| 0 | | 0.2254 | 0.3695 | 0.610 | 0.544001 |
| 1 | 3091.4 | -24.6521 | 12.4717 | -1.977 | 0.052328 |
| 2 | 3089.4 | 16.4980 | 12.8488 | 1.284 | 0.203695 |
| 3 | 2987.4 | 10.5285 | 6.9744 | 1.510 | 0.135993 |
| 4 | 2969.0 | -7.9864 | 4.2662 | -1.872 | 0.065707 |
| 5 | 2956.7 | 9.7930 | 2.8089 | 3.486 | 0.000883 |

(suite)

| Variable explicative j | Nombre d'onde σ (cm-1) | Coefficient β | Erreur Standard | Résultat de test | Probabilité |
|---|---|---|---|---|---|
| | | Estimate | Std. Error | t value | Pr (>\|t\|) |
| 6 | 2805.7 | 17.1375 | 9.4481 | 1.814 | 0.074314 |
| 7 | 1520.2 | -4.1227 | 3.0247 | -1.363 | 0.177584 |
| 8 | 1503.9 | -4.8509 | 2.6843 | -1.807 | 0.075365 |
| 9 | 1452.9 | -0.8978 | 3.3514 | -0.268 | 0.789646 |
| 10 | 1444.7 | 7.9318 | 2.8937 | 2.741 | 0.007901 |
| 11 | 1428.4 | -15.6745 | 4.9713 | -3.153 | 0.002446 |
| 12 | 1383.5 | -15.7117 | 5.3184 | -2.954 | 0.004361 |
| 13 | 1365.1 | 12.0351 | 4.7273 | 2.546 | 0.013279 |
| 14 | 1136.6 | 6.2445 | 4.4486 | 1.404 | 0.165163 |
| 15 | 1124.3 | 0.4249 | 4.2978 | 0.099 | 0.921557 |
| 16 | 1095.8 | -7.5944 | 1.6238 | -4.677 | 1.52e-05 |
| 17 | 1036.6 | 6.1430 | 2.5989 | 2.364 | 0.021092 |
| 18 | 1012.1 | -3.9852 | 3.0611 | -1.302 | 0.197555 |
| 19 | 963.1 | 2.9781 | 1.3443 | 2.215 | 0.030237 |
| 20 | 955.0 | 4.0846 | 2.1981 | 1.858 | 0.067664 |

*Tableau 5*

| Distribution des résidus en minimum, maximum et quartiles : | | | | |
|---|---|---|---|---|
| Minimum | 1er quartile | Médian | 3e quartile | Maximum |
| -0,068317 | -0,024352 | -0,004114 | 0,019129 | 0,086793 |

Écart-type résiduel : 0,03789 avec 65 degrés de liberté

Coefficient de détermination ajusté : 0,7104

F-statistique : 11,42 sur 20 et 65 degrés de liberté, P-value : 2,375e-14

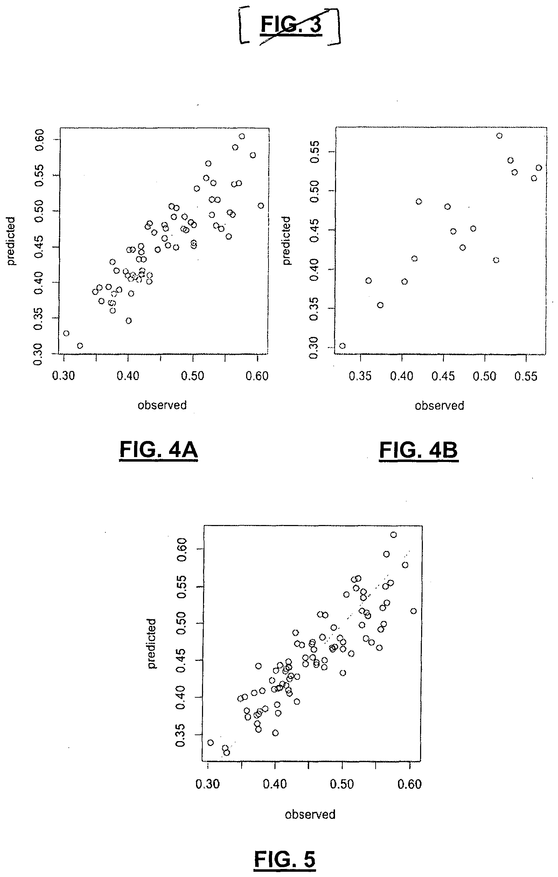

La pertinence de ce modèle à 20 nombres d'ondes est illustrée sur les figures 4A, 4B et 5.

Sur les figures 4A, 4B et 5, on a représenté la corrélation entre les valeurs de TNR prédites par le modèle et les valeurs connues pour les échantillons utilisés pour la calibration (figure 4A), pour la validation du modèle (figure 4B) et pour l'ensemble des échantillons (figure 5).

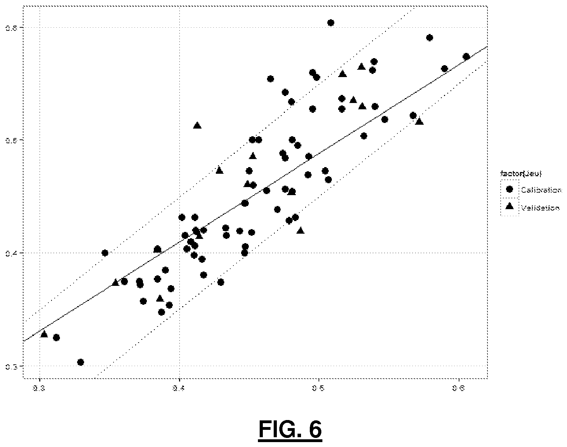

Comme on peut le voir sur la figure 6, pour 86 % des échantillons, le modèle est précis à moins de 5 points de TNR.

6.3. Exemple de mode de réalisation de l'invention

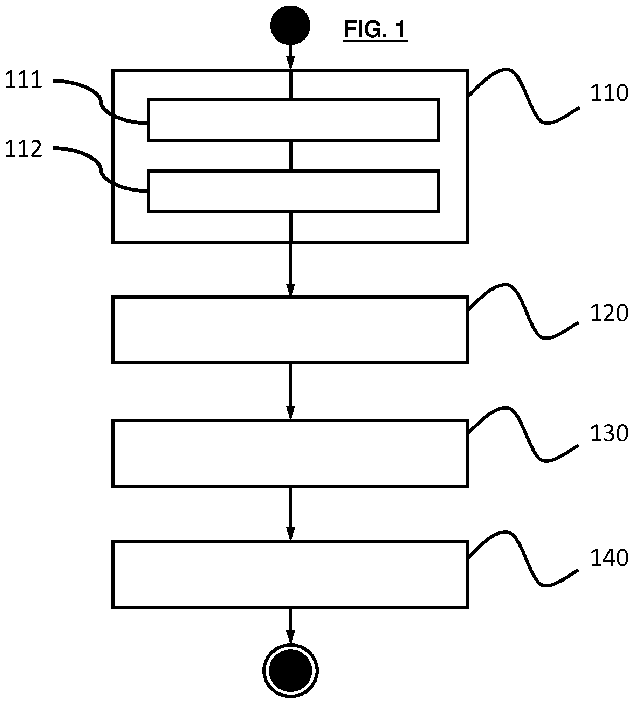

On a illustré en référence à la **figure 1**, sous forme de diagramme synoptique, les étapes d'un exemple de mode de réalisation d'un procédé de détermination de la qualité d'une semence d'un animal vertébré selon l'invention.

Lors d'une étape 110, on mesure par 3 fois le spectre d'absorption d'un culot préparé, lors d'une étape 111, à partir de deux paillettes provenant d'une semence, à l'aide d'un spectromètre de modèle FT-IR SPID et avec un capteur LS23 de DIAFIR (marque déposée).

Dans une variante de ce mode de réalisation particulier de l'invention, le spectre d'absorption peut être mesuré par transmission, par réflexion ou encore par ATR (acronyme en anglais d'"Attenuated Total Reflection").

On moyenne ensuite les 3 spectres mesurés afin d'obtenir un spectre moyenné (étape 112).

Lors d'une étape 130, on détermine à partir du spectre d'absorption moyenné une valeur de la dérivée seconde de l'absorption $X_j"$ pour n nombres d'ondes $\sigma_j$ ($1 \leq j \leq n$) caractéristiques des semences de la race de l'animal, sélectionnés lors d'une étape 120.

On calcule ensuite, lors d'une étape 140, le taux de non retour à 90 jours à partir de la loi mathématique $Y = \beta_0 + \sum_{j=1}^{n} \beta_j X_j''$ où les coefficients $\beta_o$ et $\beta_j$ (1≤j≤n) sont des constantes propres au phénotype de la race de l'animal ayant produit la semence, en utilisant les valeurs des dérivées secondes de l'absorption $X_j''$ ${}_{(j\in[1;n])}$ déterminées à l'étape 130.

Dans des variante de ce mode de réalisation particulier de l'invention, il peut être envisagé de calculer le taux de non retour à 90 jours à partir des valeurs de l'absorption $X_j$ pour n nombres d'ondes $\sigma j$ (1≤j≤n) caractéristiques des semences de la race de l'animal ou en utilisant à la fois des valeurs de l'absorption et des valeurs de la dérivée seconde de l'absorption pour les n nombres d'ondes $\sigma j$ (1≤j≤n) caractéristiques des semences de la race de l'animal.

Bien que l'invention ait été décrite en liaison avec plusieurs modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention.

Ainsi, le procédé décrit en relation avec un exemple est applicable pour toutes les races ou les espèces d'animaux vertébrés en l'adaptant au phénotype de la race ou de l'espèce via le choix des nombres d'ondes (variables explicatives) caractéristiques de la qualité de la semence de la race ou de l'espèce.

## Revendications

1. Procédé de détermination de la qualité d'une semence d'un animal vertébré non humain, comprenant les étapes suivantes :

   - mesure (110) d'au moins un spectre d'absorption d'un échantillon de ladite semence ;
   - sélection d'un nombre n, avec n ≥ 4, de nombres d'ondes $\sigma_j$ ${}_{(j\in[1;n])}$ caractéristiques des semences de la race ou de l'espèce dudit animal;
   - détermination à partir dudit ou desdits spectres d'absorption d'une valeur de l'absorption $X_j$ et/ou d'une valeur de la dérivée seconde de l'absorption $X_j''$ ${}_{(j\in[1;n])}$ pour chacun desdits n nombres d'ondes $\sigma_j$ ${}_{(j\in[1;n])}$ ;

   **caractérisé par**:
   les valeurs desdits nombres d'ondes étant dans le domaine du moyen infrarouge entre 4000 et 400 cm$^{-1}$, et un

   - calcul (140) d'un taux de non-retour Y à un nombre prédéfini de jours à partir d'une combinaison linéaire desdites valeurs de l'absorption $X_j$ et/ou de la dérivée seconde de l'absorption $X_j''$ précédemment déterminées, où, dans une étape du procédé, les valeurs des coefficients de pondération de ladite combinaison linéaire sont obtenues à partir d'un traitement des mesures des spectres d'absorption d'une pluralité d'échantillons de semence d'une population de vertébré de référence, dont les taux de non-retour sont connus.

2. Procédé de détermination de la qualité d'une semence selon la revendication 1, **caractérisé en ce que** ledit taux de non-retour Y est calculé selon la loi mathématique $Y = \beta_0 + \sum_{j=1}^{n} \beta_j X_j''$, où $X_j''$ ${}_{(j\in[1;n])}$ est la dérivée seconde normalisée de la valeur d'absorption pour le nombre d'ondes $\sigma_j$ et les coefficients de pondération $\beta_0$ et $\beta_j$ ${}_{(j\in[1;n])}$ sont des constantes.

3. Procédé de détermination de la qualité d'une semence selon l'une quelconque des revendications 1 à 2, **caractérisé en ce que** lors de ladite étape de mesure (110), au moins 2, de préférence au moins 3 spectres d'absorption d'un échantillon de ladite semence sont mesurés et **en ce que** ladite étape de détermination de valeurs de l'absorption et/ou de dérivées secondes de l'absorption comprend une étape de réalisation d'une moyenne (112) desdits spectres mesurés à partir de laquelle sont déterminés lesdites valeurs de l'absorption et/ou des dérivées secondes de l'absorption.

4. Procédé de détermination de la qualité d'une semence selon l'une quelconque des revendications 1 à 3, **caractérisé en ce que** le nombre n de nombres d'ondes $\sigma_j$ ${}_{(j\in[1;n])}$ est supérieur ou égal à 7, de préférence est supérieur ou égal à 9, encore plus préférentiellement est supérieur ou égal à 13.

5. Procédé de détermination de la qualité d'une semence selon l'une quelconque des revendications 1 à 4, **caractérisé en ce que** lesdits nombres d'ondes sont représentatifs chacun d'une molécule ou d'un ensemble de molécules choisies dans le groupe comprenant au moins :

- lipides ;
- glucides ;
- protéines ;
- acides nucléiques ;
- combinaison d'une molécule de lipide, de glucide, de protéine, ou d'acide nucléique avec au moins une autre molécule de lipide, de glucide, de protéine, ou d'acide nucléique.

6. Procédé de détermination de la qualité d'une semence selon l'une quelconque des revendications 1 à 5, **caractérisé en ce que** ledit vertébré est un taureau et le taux de non-retour est un taux de non-retour à 90 jours, ledit taureau étant issu d'une race sélectionnée dans le groupe comprenant au moins l'Abondance, la Bretonne pie noir, la Brune, la Jersiaise, la Montbéliarde, la Pie rouge des plaines, la Prim'Holstein, la Rouge flamande, la Bleue du nord, la Normande, la Salers, la Tarentaise.

7. Procédé de détermination de la qualité d'une semence selon l'une quelconque des revendications 1 à 6, **caractérisé en ce qu'**au moins un desdits nombres d'ondes $\sigma_{j\,(j\in[1;n])}$ est choisi dans la plage de nombre d'ondes 960 cm$^{-1}$ ; 1100 cm$^{-1}$], [1440 cm$^{-1}$ ; 1550 cm$^{-1}$] ou [2800 cm$^{-1}$ ; 3200 cm$^{-1}$] ou dans la bande amide B.

8. Procédé de détermination de la qualité d'une semence selon l'une quelconque des revendications 1 à 7, **caractérisé en ce que** lesdits nombres d'ondes $\sigma_{j\,(j\in[1;n])}$ sont choisis dans le groupe comprenant au moins 3165 $\pm$ 1 cm$^{-1}$, 3136 $\pm$ 1 cm$^{-1}$, 3103 $\pm$ 1 cm$^{-1}$, 3071 $\pm$ 1 cm$^{-1}$, 3026 $\pm$ 1 cm$^{-1}$, 2977 $\pm$ 1 cm$^{-1}$, 2975 $\pm$ 1 cm$^{-1}$, 1736 $\pm$ 1 cm$^{-1}$, 1689 $\pm$ 1 cm$^{-1}$, 1661 $\pm$ 1 cm$^{-1}$, 1618 $\pm$ 1 cm$^{-1}$, 1515 $\pm$ 1 cm$^{-1}$, 1448 $\pm$ 1 cm$^{-1}$, 1430 $\pm$ 1 cm$^{-1}$, 1344 $\pm$ 1 cm$^{-1}$, 1338 $\pm$ 1 cm$^{-1}$, 1336 $\pm$ 1 cm$^{-1}$, 1316 $\pm$ 1 cm$^{-1}$, 1214 $\pm$ 1 cm$^{-1}$, 1183 $\pm$ 1 cm$^{-1}$, 1103 $\pm$ 1 cm$^{-1}$, 1099 $\pm$ 1 cm$^{-1}$, 1020 $\pm$ 1 cm$^{-1}$, 975 $\pm$ 1 cm$^{-1}$.

9. Procédé de détermination de la qualité d'une semence selon la revendication 8, **caractérisé en ce que** le nombre n desdits nombres d'ondes $\sigma_{j\,(j\in[1;n])}$ sélectionnés est égal à treize et **en ce que** les valeurs desdits treize nombres d'ondes $\sigma_{j\,(j\in[1;n])}$ sélectionnés sont : 3165 $\pm$ 1 cm$^{-1}$, 3136 $\pm$ 1 cm$^{-1}$, 3103 $\pm$ 1 cm$^{-1}$, 2975 $\pm$ 1 cm$^{-1}$, 1736 $\pm$ 1 cm$^{-1}$, 1661 $\pm$ 1 cm$^{-1}$, 1515 $\pm$ 1 cm$^{-1}$, 1448 $\pm$ 1 cm$^{-1}$, 1430 $\pm$ 1 cm$^{-1}$, 1316 $\pm$ 1 cm$^{-1}$, 1214 $\pm$ 1 cm$^{-1}$, 1020 $\pm$ 1 cm$^{-1}$, 975 $\pm$ 1 cm$^{-1}$.

10. Procédé de détermination de la qualité d'une semence selon la revendication 8, **caractérisé en ce que** le nombre n desdits nombres d'ondes $\sigma_{j\,(j\in[1;n])}$ sélectionnés est égal à quinze et **en ce que** les valeurs desdits quinze nombres d'ondes $\sigma_{j\,(j\in[1;n])}$ sélectionnés sont : 3071 $\pm$ 1 cm$^{-1}$, 3026 $\pm$ 1 cm$^{-1}$, 2977 $\pm$ 1 cm$^{-1}$, 1689 $\pm$ 1 cm$^{-1}$, 1618 $\pm$ 1 cm$^{-1}$, 1515 $\pm$ 1 cm$^{-1}$, 1430 $\pm$ 1 cm$^{-1}$, 1344 $\pm$ 1 cm$^{-1}$, 1338 $\pm$ 1 cm$^{-1}$, 1336 $\pm$ 1 cm$^{-1}$, 1183 $\pm$ 1 cm$^{-1}$, 1103 $\pm$ 1 cm$^{-1}$, 1099 $\pm$ 1 cm$^{-1}$, 1020 $\pm$ 1 cm$^{-1}$, 975 $\pm$ 1 cm$^{-1}$.

11. Procédé de détermination de la qualité d'une semence selon l'une quelconque des revendications 1 à 10, **caractérisé en ce que** ladite étape de mesure (110) d'au moins un spectre d'absorption comprend une étape de préparation (111) dudit échantillon à partir de ladite semence.

12. Procédé de détermination de la qualité d'une semence selon l'une des revendications 1 à 11, **caractérisé en ce qu'**il comprend une étape de comparaison dudit taux de non-retour Y avec un seuil prédéterminé, permettant de sélectionner ladite semence pour des besoins de reproduction dans le cas où ledit taux de non-retour Y est supérieur ou égal audit seuil prédéterminé.

13. Procédé de détermination de la qualité d'une semence selon la revendication 12, **caractérisé en ce que** ledit seuil est supérieur ou égal à 0,4, de préférence supérieur ou égal à 0,5, encore plus préférentiellement supérieur ou égal à 0,6.

**Patentansprüche**

1. Verfahren zur Bestimmung der Spermaqualität eines nicht humanen Wirbeltiers, das die folgenden Schritte umfasst:

- Messen (110) mindestens eines Adsorptionsspektrums einer Probe des Spermas;
- Auswählen einer Anzahl n mit n $\geq$ 4 Wellenzahlen $\sigma_{j\,(j\in[1;n])}$, die für Sperma der Rasse oder der Art des Tieres charakteristisch sind;

- Bestimmen, auf der Basis des oder der Adsorptionsspektren, eines Absorptionswertes $X_j$ und/oder eines Wertes der zweiten Absorptionsableitung $X_j''$ $_{(j\in[1;n])}$ für jede der n Wellenzahlen $\sigma_{j\,(j\in[1;n])}$; **gekennzeichnet durch**:

die Werte der Wellenzahlen, die im mittleren Infrarotbereich zwischen 400 und 4000 cm$^{-1}$ sind, und eine Berechnung einer Non-Return-Rate $Y$ an einer vorher festgelegten Anzahl von Tagen ausgehend von einer linearen Kombination der Absorptionswerte $X_j$ und/oder der zweiten Absorptionsableitung $X_j''$, die zuvor bestimmt wurden,

wobei, bei einem Schritt des Verfahrens, die Werte der Wichtungskoeffizienten der linearen Kombination ausgehend von einer Verarbeitung der Messungen der Adsorptionsspektren einer Vielzahl von Spermaproben einer Referenzwirbeltierpopulation erhalten werden, deren Non-Return-Raten bekannt sind.

2. Verfahren zur Bestimmung der Spermaqualität nach Anspruch 1, **dadurch gekennzeichnet, dass** die Non-Return-Rate Y gemäß dem mathematischen Gesetz $Y = \beta_0 + \sum_{j=1}^n \beta_j X_j''$ berechnet wird, wobei $X_j''$ $_{(j\in[1;n])}$ die zweite Standardableitung des Absorptionswertes für die Wellenzahl $\sigma_j$ ist und die Wichtungskoeffizienten $\beta_0$ und $\beta_{j\,(j\in[1;n])}$ Konstanten sind.

3. Verfahren zur Bestimmung der Spermaqualität nach einem der Ansprüche 1 bis 2, **dadurch gekennzeichnet, dass** bei dem Messschritt mindestens 2, vorzugsweise mindestens 3 Adsorptionsspektren einer Probe des Spermas gemessen werden und dass der Bestimmungsschritt von Absorptionswerten und/oder von zweiten Absorptionsableitungen einen Realisierungsschritt eines Mittels der gemessenen Spektren umfasst, von dem ausgehend die Absorptionswerte und/oder zweite Absorptionsableitungen bestimmt sind.

4. Verfahren zur Bestimmung der Spermaqualität nach einem der Ansprüche 1 bis 3, **dadurch gekennzeichnet, dass** die Anzahl n der Wellenzahlen $\sigma_{j\,(j\in[1;n])}$ größer oder gleich 7, vorzugsweise größer oder gleich 9, noch vorzugsweiser größer oder gleich 13 ist.

5. Verfahren zur Bestimmung der Spermaqualität nach einem der Ansprüche 1 bis 4, **dadurch gekennzeichnet, dass** die Wellenzahlen jeweils für ein Molekül oder eine Molekülgruppe repräsentativ sind, die aus der Gruppe ausgewählt sind, die mindestens umfasst:

- Lipide;
- Kohlenhydrate;
- Proteine;
- Nukleinsäuren;
- Kombination eines Lipid-, Kohlenhydrat-, Protein oder Nukleinsäuremoleküls mit mindestens einem anderen Lipid-, Kohlenhydrat-, Protein oder Nukleinsäuremolekül.

6. Verfahren zur Bestimmung der Spermaqualität nach einem der Ansprüche 1 bis 5, **dadurch gekennzeichnet, dass** das Wirbeltier ein Stier ist und die Non-Return-Rate eine 90-Tage Non-Return-Rate ist, wobei der Stier zu einer Rasse gehört, die aus der Gruppe ausgewählt ist, die mindestens die Abondance, das Schwarzbunte Bretonenvieh, das Braunvieh, das Jersey-Rind, die Montbéliarde, die Pie Rouge des Plaines, die Prim'Holstein, die Rouge Flamande, die Bleue du Nord, die Normanne, die Salers, die Tarenteser umfasst.

7. Verfahren zur Bestimmung der Spermaqualität nach einem der Ansprüche 1 bis 6, **dadurch gekennzeichnet, dass** mindestens eine der Wellenzahlen $\sigma_{j\,(j\in[1;n])}$ aus dem Wellenzahlbereich 960 cm$^{-1}$; 1100 cm$^{-1}$], [1440 cm$^{-1}$; 1550 cm$^{-1}$] oder [2800 cm$^{-1}$; 3200 cm$^{-1}$] oder aus dem Amidband B ausgewählt ist.

8. Verfahren zur Bestimmung der Spermaqualität nach einem der Ansprüche 1 bis 7, **dadurch gekennzeichnet, dass** die Wellenzahlen $\sigma_{j\,(j\in[1;n])}$ aus der Gruppe ausgewählt sind, die mindestens 3165 ± 1 cm$^{-1}$, 3136 ± 1 cm$^{-1}$, 3103 ± 1 cm$^{-1}$, 3071 ± 1 cm$^{-1}$, 3026 ± 1 cm$^{-1}$, 2977 ± 1 cm$^{-1}$, 2975 ± 1 cm$^{-1}$, 1736 ± 1 cm$^{-1}$, 1689 ± 1 cm$^{-1}$, 1661 ± 1 cm$^{-1}$, 1618 ± 1 cm$^{-1}$, 1515 ± 1 cm$^{-1}$, 1448 ± 1 cm$^{-1}$, 1430 ± 1 cm$^{-1}$, 1344 ± 1 cm$^{-1}$, 1338 ± 1 cm$^{-1}$, 1336 ± 1 cm$^{-1}$, 1316 ± 1 cm$^{-1}$, 1214 ± 1 cm$^{-1}$, 1183 ± 1 cm$^{-1}$, 1103 ± 1 cm$^{-1}$, 1099 ± 1 cm$^{-1}$, 1020 ± 1 cm$^{-1}$, 975 ± 1 cm$^{-1}$ umfasst.

**9.** Verfahren zur Bestimmung der Spermaqualität nach Anspruch 8, **dadurch gekennzeichnet, dass** die Anzahl n der ausgewählten Wellenzahlen $\sigma_{j\,(j\in[1;n])}$ gleich dreizehn ist und dass die Werte der dreizehn ausgewählten Wellenzahlen $\sigma_{j\,(j\in[1;n])}$ $3165 \pm 1$ cm$^{-1}$, $3136 \pm 1$ cm$^{-1}$, $3103 \pm 1$ cm$^{-1}$, $2975 \pm 1$ cm$^{-1}$, $1736 \pm 1$ cm$^{-1}$, $1661 \pm 1$ cm$^{-1}$, $1515 \pm 1$ cm$^{-1}$, $1448 \pm 1$ cm$^{-1}$, $1430 \pm 1$ cm$^{-1}$, $1316 \pm 1$ cm$^{-1}$, $1214 \pm 1$ cm$^{-1}$, $1020 \pm 1$ cm$^{-1}$, $975 \pm 1$ cm$^{-1}$ sind.

**10.** Verfahren zur Bestimmung der Spermaqualität nach Anspruch 8, **dadurch gekennzeichnet, dass** die Anzahl n der ausgewählten Wellenzahlen $\sigma_{j\,(j\in[1;n])}$ gleich fünfzehn ist und dass die Werte der fünfzehn ausgewählten Wellenzahlen $\sigma_{j\,(j\in[1;n])}$ $3071 \pm 1$ cm$^{-1}$, $3026 \pm 1$ cm$^{-1}$, $2977 \pm 1$ cm$^{-1}$, $1689 \pm 1$ cm$^{-1}$, $1618 \pm 1$ cm$^{-1}$, $1515 \pm 1$ cm$^{-1}$, $1430 \pm 1$ cm$^{-1}$, $1344 \pm 1$ cm$^{-1}$, $1338 \pm 1$ cm$^{-1}$, $1336 \pm 1$ cm$^{-1}$, $1183 \pm 1$ cm$^{-1}$, $1103 \pm 1$ cm$^{-1}$, $1099 \pm 1$ cm$^{-1}$, $1020 \pm 1$ cm$^{-1}$, $975 \pm 1$ cm$^{-1}$ sind.

**11.** Verfahren zur Bestimmung der Spermaqualität nach einem der Ansprüche 1 bis 10, **dadurch gekennzeichnet, dass** der Messschritt (110) von mindestens einem Adsorptionsspektrum einen Vorbereitungsschritt (111) der Probe aus dem Sperma umfasst.

**12.** Verfahren zur Bestimmung der Spermaqualität nach einem der Ansprüche 1 bis 11, **dadurch gekennzeichnet, dass** es einen Vergleichsschritt der Non-Return-Rate Y mit einem vorher festgelegten Grenzwert umfasst, der erlaubt, das Sperma für Reproduktionsbedarfe in dem Fall auszuwählen, wenn die Non-Return-Rate Y über dem vorher festgelegten Grenzwert liegt oder diesem entspricht.

**13.** Verfahren zur Bestimmung der Spermaqualität nach Anspruch 12, **dadurch gekennzeichnet, dass** der Grenzwert größer oder gleich 0,4, vorzugsweise größer oder gleich 0,5, noch vorzugsweiser größer oder gleich 0,6 ist.

**Claims**

**1.** A method for determining the quality of a semen of a non-human vertebrate animal comprising the following steps:

- measuring (110) at least one absorption spectrum of a sample of said semen;
- selecting a number n, with $n \geq 4$, of wave numbers $\sigma_{j\,(j\in[1;n])}$ which are characteristic of the semens of the breed or of the species of said animal;
- determining from said at least one absorption spectrum of a value of the absorption $X_j$ and/or a value of the second derivative of the absorption $X_j"_{(j\,\in\,[1;\,n])}$ for each of said n wave numbers $\sigma_{j\,(j\in[1;n])}$;

wherein said wave numbers values being in the mid-infrared in between 4000 cm$^{-1}$ and 400 cm$^{-1}$, the method comprises a step of calculation a non-return rate Y at a predefined number of days from a linear combination of said absorption values $X_j$ and/or of the second derivative of the absorption $X_j"$ previously determined, and wherein, in a step of the method, values of the weighting coefficients of the linear combination are obtained from a processing of measurements of the absorption spectra of a plurality of semen samples from a population of reference vertebrate, of which the non-return rates are known.

**2.** The Method for determining the quality of a semen according to claim 1, **characterised in that** said non-return rate Y is calculated according to the mathematical law $Y = \beta_0 + \sum_{j=1}^{n} \beta_j X_j''$, wherein $X_j"_{(j\in[1;n])}$ is the normalized second derivative of the absorption value for the wave number $\sigma_j$ and the weighting coefficients $\beta_0$ and $\beta_{j\,(j\in[1;n])}$ are constants.

**3.** The method for determining the quality of a semen according to any one of claims 1 to 2, **characterised in that** in said measuring step (110) at least 2, preferably at least 3 absorption spectra of a sample of said semen are measured and **in that** said step of determining values of absorption and/or of second derivatives of the absorption comprises a step of averaging (112) said measured spectra from which said values of the absorption and/or the second derivatives of the absorption are determined.

**4.** The method for determining the quality of a semen according to any one of claims 1 to 3, **characterized in that** the number n of wave numbers $\sigma_{j\,(j\in[1;n])}$ is greater than or equal to 7 preferably, is greater than or equal to 9, still more preferably is greater than or equal to 13.

5. The method for determining the quality of a semen according to any one of claims 1 to 4, **characterized in that** said wave numbers are each representative of a molecule or set of molecules selected from the group consisting of at least:

   - lipids;
   - carbohydrates;
   - proteins;
   - nucleic acids;
   - a combination of a lipid, carbohydrate, protein, or nucleic acid molecule with at least one other lipid, carbohydrate, protein, or nucleic acid molecule.

6. The method for determining the quality of a semen according to any one of claims 1 to 5, **characterised in that** said vertebrate is a bull and the non-return rate is a 90-day non-return rate, said bull coming from a breed selected from the group comprising at least one of the following breeds: Abondance, Béarnaise, Bordelaise, Bretonne pie noir, Brune, Froment du Léon, Jersiaise, Montbéliarde, Pie rouge des plaines, Prim'Holstein, Rouge flamande, Bleue du nord, Normande, Salers, Tarentaise.

7. The method for determining the quality of a semen according to any one of claims 1 to 6, **characterized in that** at least one of said wave numbers $\sigma_{j\ (j\in[1;n])}$ is selected in the wave number range [960 cm$^{-1}$; 1100 cm$^{-1}$], [1440 cm$^{-1}$; 1550 cm$^{-1}$] or [2800 cm$^{-1}$; 3200 cm$^{-1}$] or in the amide B band.

8. The method for determining the quality of a semen according to any one of claims 1 to 7, **characterized in that** said wave numbers $\sigma_{j\ (j\in[1;n])}$ are selected from the group comprising at least 3165 $\pm$ 1 cm$^{-1}$, 3136 $\pm$ 1 cm$^{-1}$, 3103 $\pm$ 1 cm$^{-1}$, 3071 $\pm$ 1 cm$^{-1}$, 3026 $\pm$ 1 cm$^{-1}$, 2977 $\pm$ 1 cm$^{-1}$, 2975 $\pm$ 1 cm$^{-1}$, 1736 $\pm$ 1 cm$^{-1}$, 1689 $\pm$ 1 cm$^{-1}$, 1661 $\pm$ 1 cm$^{-1}$, 1618 $\pm$ 1 cm$^{-1}$, 1515 $\pm$ 1 cm$^{-1}$, 1448 $\pm$ 1 cm$^{-1}$, 1430 $\pm$ 1 cm$^{-1}$, 1344 $\pm$ 1 cm$^{-1}$, 1338 $\pm$ 1 cm$^{-1}$, 1336 $\pm$ 1 cm$^{-1}$, 1316 $\pm$ 1 cm$^{-1}$, 1214 $\pm$ 1 cm$^{-1}$, 1183 $\pm$ 1 cm$^{-1}$, 1103 $\pm$ 1 cm$^{-1}$, 1099 $\pm$ 1 cm$^{-1}$, 1020 $\pm$ 1 cm$^{-1}$, 975 $\pm$ 1 cm$^{-1}$.

9. The method for determining the quality of a semen according to claim 8, **characterized in that** the number n of said selected wave numbers $\sigma_{j\ (j\in[1;n])}$ is equal to thirteen and **in that** the values of said thirteen selected wave numbers $\sigma_{j\ (j\in[1;n])}$ are: 3165 $\pm$ 1 cm$^{-1}$, 3136 $\pm$ 1 cm$^{-1}$, 3103 $\pm$ 1 cm$^{-1}$, 2975 $\pm$ 1 cm$^{-1}$, 1736 $\pm$ 1 cm$^{-1}$, 1661 $\pm$ 1 cm$^{-1}$, 1515 $\pm$ 1 cm$^{-1}$, 1448 $\pm$ 1 cm$^{-1}$, 1430 $\pm$ 1 cm$^{-1}$, 1316 $\pm$ 1 cm$^{-1}$, 1214 $\pm$ 1 cm$^{-1}$, 1020 $\pm$ 1 cm$^{-1}$, 975 $\pm$ 1 cm$^{-1}$.

10. The method for determining the quality of a semen according to claim 8, **characterised in that** the number n of said selected wave numbers $\sigma j_{\ (j\in[1;n])}$ is equal to fifteen and **in that** the values of said fifteen selected wave numbers $_j\ \sigma j_{\in[1;n])}$ are: 3071 $\pm$ 1 cm$^{-1}$, 3026 $\pm$ 1 cm$^{-1}$, 2977 $\pm$ 1 cm$^{-1}$, 1689 $\pm$ 1 cm$^{-1}$, 1618 $\pm$ 1 cm$^{-1}$, 1515 $\pm$ 1 cm$^{-1}$, 1430 $\pm$ 1 cm$^{-1}$, 1344 $\pm$ 1 cm$^{-1}$, 1338 $\pm$ 1 cm$^{-1}$, 1336 $\pm$ 1 cm$^{-1}$, 1183 $\pm$ 1 cm$^{-1}$, 1103 $\pm$ 1 cm$^{-1}$, 1099 $\pm$ 1 cm$^{-1}$, 1020 $\pm$ 1 cm$^{-1}$, 975 $\pm$ 1 cm$^{-1}$.

11. The method for determining the quality of a semen according to any one of claims 1 to 10, **characterised in that** said step of measuring (110) at least one absorption spectrum comprises a step of preparing (111) said sample from said semen.

12. The method for determining the quality of a semen according to any one of claims 1 to 11, **characterized in that** it comprises a step of comparing said non-return rate Y with a predetermined threshold, for selecting said semen for reproduction purposes in case where said non-return rate Y is greater than or equal to said predetermined threshold.

13. The method for determining the quality of a semen according to claim 12, **characterised in that** the said threshold is greater than or equal to 0.4 , preferably greater than or equal to 0.5 , even more preferably greater than or equal to 0.6.

FIG. 1

**FIG. 2**

[ FIG. 3 ]

FIG. 3

FIG. 4A

FIG. 4B

FIG. 5

**FIG. 6**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2958403
- WO 2013017324 A
- FR 1450661

- WO 2013064159 A1
- CN 103698310 A

**Littérature non-brevet citée dans la description**

- **DEJARNETTE JM ; AMANN RP.** Understanding estimates of AI sire fertility: From A to Z. *23rd Technical Conference on Artificial Insemination & Reproduction, Milwaukee, WI,* 2010
- WHO laboratory manual for the examination and processing of human semen. World Health Organization, 2010
- **DE LONGE C.** Semen analysis: looking for an upgrade in class. *Fertil Steril,* 2012, vol. 97, 260-266
- **KASTELIC JP ; THUNDATHIL JC.** Breeding soundness évaluation and semen analysis for predicting bull fertility. *Reprod Domest Anim Zuchthyg,* 2008, vol. 43 (2), 368-373
- **MOCÉ E ; GRAHAM JK.** In vitro évaluation of sperm quality. *Anim Reprod Sci,* 2008, vol. 105, 104-118
- **DACHEUX J-L ; DACHEUX F.** New insights into epididymal function in relation to sperm maturation. *Reprod Camb Engl,* 2014, vol. 147, R27-42
- **HOLT WV ; VAN LOOK KJW.** Concepts in sperm heterogeneity, sperm sélection and sperm compétition as biological foundations for laboratory tests of semen quality. *Reprod Camb Engl,* 2004, vol. 127, 527-535
- **RODRIGUEZ-MARTINEZ H.** Can we increase the estimated value of semen assessment?. *Reprod Domest Anim Zuchthyg,* 2006, (2), 2-10
- **PETRUNKINA AM ; VOLKER G ; BRANDT H ; TÖPFER-PETERSEN E ; WABERSKI D.** Functional significance of responsiveness to capacitating conditions in boar spermatozoa. *Theriogenology,* 2005, vol. 64, 1766-1782
- **PETRUNKINA AM ; VOLKER G ; BRANDT H ; TÖPFER-PETERSEN E ; WABERSKI D.** Functional significance of responsiveness to capacitating conditions in boar spermatozoa. *Theriogenology,* 2005, vol. 64, 1766-1782
- **AMANN RP ; HAMMERSTEDT RH.** In vitro évaluation of sperm quality: an opinion. *J Androl,* 1993, vol. 14, 397-406

- **GRAHAM JK ; MOCÉ E.** Fertility évaluation of frozen/thawed semen. *Theriogenology,* 2005, vol. 64, 492-504
- **JANUSKAUSKAS A ; JOHANNISSON A ; SÖDERQUIST L ; RODRIGUEZ-MARTINEZ H.** Assessment of sperm characteristics post-thaw and response to calcium ionophore in relation to fertility in Swedish dairy AI bulls. *Theriogenology,* 2000, vol. 53, 859-875
- **GRAHAM JK.** Assessment of sperm quality: a flow cytometric approach. *Anim Reprod Sci,* 2001, vol. 68, 239-247
- **EGEA RR ; PUCHALT NG ; ESCRIVÁ MM ; VARGHESE AC.** OMICS: « Current and future perspectives in reproductive medicine and technology. *J Hum Reprod Sci,* 2014, vol. 7, 73-92
- **ROBERT C.** Challenges of functional genomics applied to farm animal gametes and pre-hatching embryos. *Theriogenology,* 2008, vol. 70, 1277-1287
- **JONES JJ ; STUMP MJ ; FLEMING RC ; LAY JO ; WILKINS CL.** Strategies and data analysis techniques for lipid and phospholipid chemistry elucidation by intact cell MALDI-FTMS. *J Am Soc Mass Spectrom,* 2004, vol. 15, 1665-1674
- **LABAS V ; SPINA L ; BELLEANNEE C ; TEIXEIRA-GOMES A-P ; GARGAROS A ; DACHEUX F ; DACHEUX J-L.** Analysis of epididymal sperm maturation by MALDI profiling and top-down mass spectrometry. *J Proteomics,* 2015, vol. 113, 226-243
- **HELM D ; LABISCHINSKI H ; SCHALLEHN G ; NAUMANN D.** Classification and identification of bacteria by Fourier-transform infrared spectroscopy. *J Gen Microbiol,* 1991, vol. 137, 69-79
- **STAMM I ; HAILER M ; DEPNER B ; KOPP PA ; RAU J.** Yersinia enterocolitica in diagnostic fecal samples from European dogs and cats: identification by fourier transform infrared spectroscopy and matrix-assisted laser desorption ionization-time of flight mass spectrometry. *J Clin Microbiol,* 2013, vol. 51, 887-893

- **BACKHAUS J ; MUELLER R ; FORMANSKI N ; SZLAMA N ; MEERPOHL H-G ; EIDT M ; BUGERT P.** Diagnosis of breast cancer with infrared spectroscopy from sérum samples. *Vib Spectrosc,* 2010, vol. 52, 173-177
- **KALLENBACH-THIELTGES A ; GROßERÜSCHKAMP F ; MOSIG A ; DIEM M ; TANNAPFEL A ; GERWERT K.** Immunohistochemistry, histopathology and infrared spectral histopathology of colon cancer tissue sections. *J Biophotonics,* 2013, vol. 6, 88-100
- **LEWIS PD ; LEWIS KE ; GHOSAL R ; BAYLISS S ; LLOYD AJ ; WILLS J ; GODFREY R ; KLOER P ; MUR LAJ.** Evaluation of FTIR spectroscopy as a diagnostic tool for lung cancer using sputum. *BMC Cancer,* 2010, vol. 10, 640
- **CANVIN JMG ; BERNATSKY S ; HITCHON CA ; JACKSON M ; SOWA MG ; MANSFIELD JR ; EYSEL HH ; MANTSCH HH ; EL-GABALAWY HS.** Infrared spectroscopy: shedding light on synovitis in patients with rheumatoid arthritis. *Rheumatol Oxf Engl,* 2003, vol. 42, 76-82
- **DE CARVALHO BMA ; DE CARVALHO LM ; REIS COIMBRA JS DOS ; MINIM LA ; DE SOUZA BARCELLOS E ; DA SILVA JÚNIOR WF ; DETMANN E ; DE CARVALHO GGP.** Rapid détection of whey in milk powder samples by spectrophotometric and multivariate calibration. *Food Chem,* 2015, vol. 174, 1-7
- **MALLIDIS C ; SANCHEZ V ; WISTUBA J ; WUEBBELING F ; BURGER M ; FALLNICH C ; SCHLATT S.** Raman microspectroscopy: shining a new light on reproductive medicine. *Hum Reprod Update,* 2014, vol. 20, 403-414
- **MALLIDIS C ; WISTUBA J ; BLEISTEINER B ; DAMM OS ; GROSS P ; WÜBBELING F ; FALLNICH C ; BURGER M ; SCHLATT S.** In situ visualization of damaged DNA in human sperm by Raman microspectroscopy. *Hum Reprod Oxf Engl,* 2011, vol. 26, 1641-1649
- **MALLIDIS C ; SCHLATT S ; WISTUBA J ; FALLNICH C ; GROSS P ; BURGER M ; WUEBBELING F.** *Means and methods for assessing sperm nuclear DNA structure.*
- **HUANG Z ; CHEN X ; CHEN Y ; CHEN J ; DOU M ; FENG S ; ZENG H ; CHEN R.** Raman spectroscopic characterization and differentiation of seminal plasma. *J Biomed Opt,* 2011, vol. 16, 110501-1105013
- **PEREZ-MARIN C C et al.** Assessment of porcine semen concentration by near InfraRed Spectroscopy (NIRS). *REPRODUCTION IN DOMESTIC ANIMALS,* 01 Septembre 2007, vol. 42 (2), 92